# EUROPEAN PATENT SPECIFICATION

(11) **EP 2 188 911 B1**
(45) Date of publication and mention of the grant of the patent: **12.10.2011**
(21) Application number: 08795298.2
(22) Date of filing: 13.08.2008
(51) Int. Cl.: H04W 52/40, H04W 52/54

(54) **TRANSFER OF UPLINK POWER CONTORL PARAMETERS DURING HANDOVERS**
ÜBERTRAGUNG VON UPLINK-LEISTUNGSSTEUERPARAMETERN WÄHREND WEITERLEITUNGEN
TRANSFERT DE PARAMÈTRES DE CONTRÔLE DE PUISSANCE SUR VOIE MONTANTE LORS DE TRANSFERTS INTERCELLULAIRES

(30) Priority: 14.08.2007 US 893029
(43) Date of publication of application: 26.05.2010
(73) Proprietor: Alcatel Lucent, 75007 Paris (FR)
(72) Inventor: RAO, Anil, M., Cedar Knolls, NJ 07927 (US)
(74) Representative: Schmidt, Werner Karl
(86) International application number: PCT/US2008/009698
(87) International publication number: WO 2009/023228

(56) References cited:
- EP-A- 0 883 251
- US-B1- 6 278 879

## Description

### FIELD OF THE INVENTION

The present invention relates generally to wireless communication systems, and more particularly to handovers in communication systems.

### BACKGROUND OF THE INVENTION

A mobile unit, sometimes referred to as user equipment or a communication unit, can move from the coverage area of a first (source) base station to the coverage area of a second (target) base station. The process of transferring control from the first base station to the second base station is often referred to as handover or handoff.

Although handover allows a mobile unit the ability to travel within, and even outside, of a cellular network, there are problems that arise during handover. Handover problems include delays in the connection between the mobile unit and the second base station and a mobile unit transmitting at an incorrect power level when moving to the target base station.

A first solution of the prior art involves waiting for 40-100 ms to obtain the proper power control parameters from the target base station. However, this causes a loss in throughput and/or dropped packets for latency sensitive applications such as gaming over IP and voice over IP. A second solution is to simply have the mobile unit transmit at the power setting that it was using at the source base station. However, given the potential difference in uplink interference conditions at the different base stations as well as possible differences in uplink power control parameters, the mobile unit will not be transmitting at the proper power level; too high a power level results in excess interference and hence reduced system performance, whereas too low a power level results in quality of service degradation for the user.

Two key features of the LTE (Long Term Evolution) of 3GPP that are significantly different compared to the legacy UMTS systems are that soft handover is not supported in the uplink and that open loop power control will be used in the uplink. Soft handover not being supported implies that the mobile unit must disconnect from the current serving base station and then reconnect to the target base station and restart its transmission.

Using open link power control in the uplink Implies that the mobile unit autonomously adjusts its transmission power in order to achieve a specific target signal to interference plus noise ratio (SINR) at the serving base station. Therefore in 3GPP LTE systems a potentially lengthy procedure needs to be performed before the UE is able to transmit at the proper power level after switching from its current serving base station to the target base station.

The consequence of delaying transmission until the proper transmission power can be determined is extra latency, leading to a loss in throughput. For latency sensitive applications like voice over IP (VoIP) or Video Telephony (VT), this extra latency may lead to dropped voice packets, causing unacceptable voice quality during cell switch. It is also possible that the UE simply transmits at the power level it was transmitting with to the source base station even after it switches to the target base station, but this can cause unnecessarily high levels of interference if the power levels is too high, or result in quality of service (QoS) degradation if the power level is too low.

Therefore, a need exists for a method of performing handover that does not cause delays in transmission upon handover, dropped voice packets, unacceptable voice quality, or increased levels of interference.

EP0883251A2 relates to power control of mobile station transmission during handoff in a cellular system. A method and apparatus for controlling the transmission power of the mobile station during handoff of a call between base stations and cellular system is provided. The method and apparatus allows the desired transmission power of the mobile station on the assigned traffic channel of a target base station to be estimated and allows the transmission power of the mobile station to be adjusted over a range of power values.

### BRIEF SUMMARY OF THE INVENTION

The present invention provides a solution to the problems of the prior art by transmitting uplink power control parameters to a mobile unit prior to handover completion.

In an exemplary embodiment of the present invention, transfer uplink power control parameters from the target base station are sent to the mobile unit by the source base station during the handover procedure. This allows the mobile unit to have all parameters necessary to immediately begin transmitting at the proper power setting to the new target base station, avoiding the issues of either waiting to obtain the proper power control parameters or transmitting with an incorrect power setting.

The present invention thereby eliminates the need for a mobile unit to wait for power control parameters to be broadcast in the new target base station before transmitting at the proper uplink power level. This saves from 40 ms to 100 ms of delay during handover to a new base station. This allows the mobile unit to transmit at the proper power setting immediately after synchronizing to the new base station, which avoids QoS degradation or generation of excess interference which would be caused by the mobile transmitting with an incorrect power setting or delaying transmission until the power control parameters are available.

### BRIEF DESCRIPTION OF THE SEVERAL VIEWS OF THE DRAWINGS

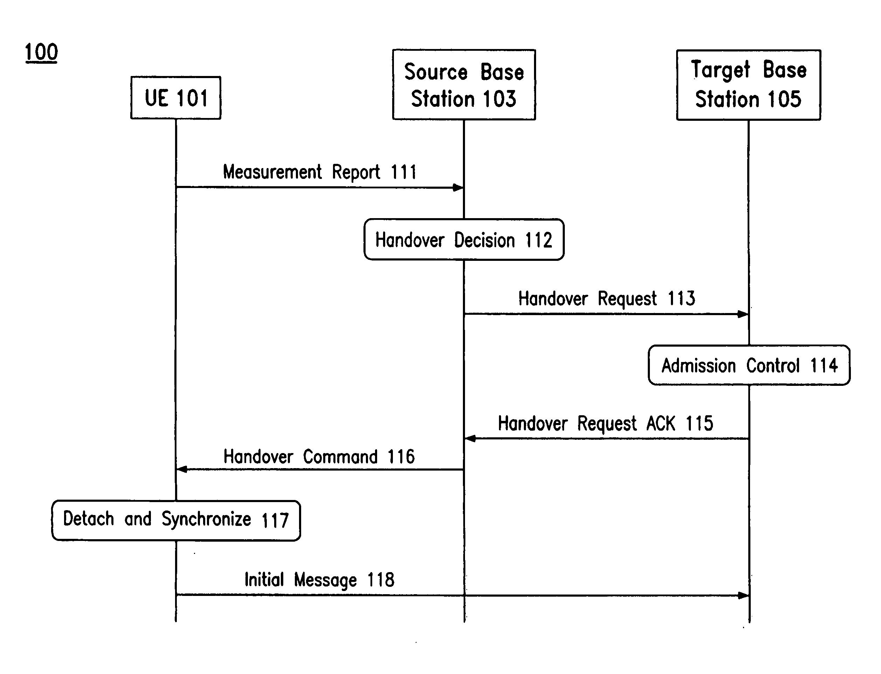

The figure depicts a message flow between a communication unit, a source base station, and a target base station in accordance with an exemplary embodiment of the present invention.

### DETAILED DESCRIPTION OF THE INVENTION

The figure depicts a message flow 100 between a communication unit, also referred to as a mobile unit or user equipment (UE) 101, a source base station 103, and a target base station 105 in accordance with an exemplary embodiment of the present invention. The figure depicts an exemplary embodiment of the present invention wherein uplink power control settings are transferred from the target base station to the mobile unit via the source base station during the handover procedure. This allows the mobile unit to transmit at the proper power setting immediately after connecting to the target base station, saving 40-100 ms in delay.

UE 101 sends measurement report message 111 to source base station 103. Measurement report message 111 preferably includes parameters that are useful in assisting source base station 103 in deciding if UE 101 should be handed off to another base station. In an exemplary embodiment, measurement report message 111 includes the path loss that UE 101 measures from source base station 103 and the path loss that UE 101 measures from target base station 105. Additionally, measurement report message 111 preferably includes an identification of target base station 105.

Source base station 103 evaluates the measurements sent in measurement report message 111 and decides (112) whether UE 101 should be transferred to target base station 105. This handover decision may, for example, make a handover determination when the path loss between UE 101 and target base station 105 is sufficiently smaller than the path loss between UE 101 and source base station 103. In this example, if the difference in path loss exceeds a predetermined threshold, source base station 103 will make a determination that UE 101 should be handed over to target base station 105.

If source base station 103 determines that a handover of UE 101 to target base station 105 is warranted, source base station 103 sends handover request message 113 to target base station 105. In an exemplary embodiment, handover request message 113 is sent via an X2 interface that preferably connects base stations within the communication network. In an exemplary embodiment, handover request message 113 also includes information relating to the connection of UE 101, such as Quality of Service (QoS) attributes.

Target base station 105 performs admission control (114) to evaluate whether it can support UE 101. In an exemplary embodiment, target base station 105 utilizes current loading and availability of resources at target base station 105 to determine if it will can successfully handle UE 101.

Target base station 105 sends handover request ACK message 115 to source base station 103. If target base station 105 determines that it will accept the handover of UE 101, handover request ACK message 115 includes the uplink power control parameters that it sends in its broadcast message. For example, handover request ACK message 115 preferably includes the uplink interference level at target base station 105 and the target Signal to Interference-plus-Noise Ratio (SINR) at target base station 105.

Source base station 103 sends handover command message 116 to UE 101. Handover command message 116 informs UE 101 to connect to target base station 105 and includes the uplink power control parameters for target base station 105.

UE 101 then detaches from source base station 103 and synchronizes (117) with target base station 105. The synchronization preferably includes a random access procedure with target base station 105. This procedure gets the proper timing for UE 101 in the uplink. In an exemplary embodiment, the synchronization procedure leads to an interruption of transmission on the order of 30 to 40 milliseconds, which is significantly less than the service interruption in the prior art.

UE 101 transmits initial message 118 at the correct power level by using the uplink power control parameters for target base station 105 that UE 101 received in handover command message 116.

While this invention has been described in terms of certain examples thereof, it is not intended that it be limited to the above description, but rather only to the extent set forth in the claims that follow.

## Claims

1. A method for performing handover of a mobile unit from a source base station to a target base station, the method comprising: initiating a handover request of a mobile unit with a source base station; transmitting uplink power control parameters associated with a target base station to a source base station prior to completing handover of the mobile unit to the target base station; and transmitting the uplink power control parameters from the source base station to a mobile unit, wherein the step of transmitting uplink power control parameters associated with a target base station to a source base station comprises transmitting uplink power control parameters associated with a target base station to a source base station as part of a handover request confirm message.

2. A method for performing handover of a mobile unit from a source base stations to a target base station in accordance with claim 1 , wherein the step of transmitting the uplink power control parameters from the source base station to a mobile unit comprises transmitting the uplink power control parameters from the source base station to a mobile unit as part of a handover command.

3. A method for performing handover of a mobile unit from a source base station to a target base station in accordance with claim 1, wherein the uplink power control parameters include an uplink interference level at the target base station,

4. A method for performing handover of a mobile unit from a source base station to a target base station in accordance with claim 1 , wherein the uplink power control parameters include a target signal to interference plus noise ratio, SINR, at the target base station.

5. A method for performing handover of a mobile unit from a source base station to a target base station in accordance with claim 1, wherein the uplink power control parameters include a fractional power control parameters at the target base station.

6. A method for performing handover of a mobile unit from a source base station to a target base station in accordance with claim 1 , the method further comprising the step of transmitting a path loss measurement between the mobile unit and the target base station from the mobile unit.

7. A method for performing handover of a mobile unit from a source base station to a target base station in accordance with claim 6, further comprising the step of utilizing the path loss measurement when transmitting between the mobile unit and the target base station.

8. A method for performing handover of a mobile unit from a source base station to a target base station in accordance with claim 1 , the method further comprising the step of transmitting from the mobile unit to the target base station using the uplink power control parameters.

9. A method for performing handover of a mobile unit from a source base station to a target base station in accordance with claim 1, the method further comprising the step of transmitting from the mobile unit to the target base station using the uplink power control parameters and a path loss measurement between the mobile unit and the target base station.

## Patentansprüche

1. Verfahren zum Durchführen von Handover einer mobilen Einheit von einer Quell-Basisstation an eine Ziel-Basisstation, wobei das Verfahren umfasst: Initiierten einer Handover-Anforderung einer mobilen Einheit mit einer Quell-Basisstation; Übertragen von mit einer Ziel-Basisstation assoziierten Uplink-Leistungssteuerparametern an eine Quell-Basisstation vor Durchführen des Handovers der mobilen Einheit an die Ziel-Basisstation; und Übertragen der Uplink-Leistungssteuerparameter von der Quell-Basisstation an eine mobile Einheit, wobei der Schritt des Übertragens von mit einer Ziel-Basisstation assoziierten Uplink-Leistungssteuerparametern an eine Quell-Basisstation das Übertragen von mit einer Ziel-Basisstation assoziierten Uplink-Leistungssteuerparametern an eine Quell-Basisstation als Teil einer Handoveranforderungs-Bestätigungsnachricht umfasst.

2. Verfahren zum Durchführen von Handover einer mobilen Einheit von einer Quell-Basisstation an eine Ziel-Basisstation nach Anspruch 1, wobei der Schritt des Übertragens der Uplink-Leistungssteuerparameter von der Quell-Basisstation an eine mobile Einheit das Übertragen der Uplink-Leistungssteuerparameter von der Quell-Basisstation an eine mobile Einheit als Teil eines Handover-Befehls umfasst.

3. Verfahren zum Durchführen von Handover einer mobilen Einheit von einer Quell-Basisstation an eine Ziel-Basisstation nach Anspruch 1, wobei die Uplink-Leistungssteuerparameter einen Uplink-Störpegel an der Ziel-Basisstation umfassen.

4. Verfahren zum Durchführen von Handover einer mobilen Einheit von einer Quell-Basisstation an eine Ziel-Basisstation nach Anspruch 1, wobei die Uplink-Leistungssteuerparameter ein Ziel-Signal-zu-Störungs-plus-Rauschverhältnis, SINR, an der Ziel-Basisstation umfassen.

5. Verfahren zum Durchführen von Handover einer mobilen Einheit von einer Quell-Basisstation an eine Ziel-Basisstation nach Anspruch 1, wobei die Uplink-Leistungssteuerparameter einen Leistungssteuer-Bruchparameter an der Ziel-Basisstation umfassen.

6. Verfahren zum Durchführen von Handover einer mobilen Einheit von einer Quell-Basisstation an eine Ziel-Basisstation nach Anspruch 1, wobei das Verfahren weiterhin den Schritt des Übertragens einer Pfadverlustmessung zwischen der mobilen Einheit an der Ziel-Basisstation von der mobilen Einheit umfasst.

7. Verfahren zum Durchführen von Handover einer mobilen Einheit von einer Quell-Basisstation an eine Ziel-Basisstation nach Anspruch 6, weiterhin umfassend den Schritt des Verwendens der Pfadverlustmessung bei der Übertragung zwischen der mobilen Einheit und der Ziel-Basisstation.

8. Verfahren zum Durchführen von Handover einer mobilen Einheit von einer Quell-Basisstation an eine Ziel-Basisstation nach Anspruch 1, wobei das Verfahren weiterhin den Schritt des Übertragens von der mobilen Einheit an die Ziel-Basisstation unter Verwendung der Uplink-Leistungssteuerparameter umfasst.

9. Verfahren zum Durchführen von Handover einer mobilen Einheit von einer Quell-Basisstation an eine Ziel-Basisstation nach Anspruch 1, wobei das Verfahren weiterhin den Schritt des Übertragens von der mobilen Einheit an die Ziel-Basisstation unter Verwendung der Uplink-Leistungssteuerparameter und einer Pfadverlustmessung zwischen der mobilen Einheit und der Ziel-Basisstation umfasst.

## Revendications

1. Procédé d'exécution d'un transfert d'une unité mobile entre une station de base source et une station de base cible, le procédé comprenant les étapes suivantes :
initier une demande de transfert d'une unité mobile avec une station de base source ; transmettre des paramètres de commande de puissance en liaison montante, associés à une station de base cible, à une station de base source avant de terminer un transfert de l'unité mobile vers la station de base cible ; et
transmettre les paramètres de commande de puissance en liaison montante entre la station de base source et une unité mobile, dans lequel l'étape de transmission de paramètres de commande de puissance en liaison montante, associés à une station de base cible, à une station de base source comprend la transmission de paramètres de commande de puissance en liaison montante, associés à une station de base cible, à une station de base source en tant que partie d'un message de confirmation de demande de transfert.

2. Procédé d'exécution d'un transfert d'une unité mobile entre une station de base source et une station de base cible selon la revendication 1, dans lequel l'étape de transmission des paramètres de commande de puissance en liaison montante entre la station de base source et une unité mobile comprend la transmission des paramètres de commande de puissance en liaison montante entre la station de base source et une unité mobile en tant que partie d'une commande de transfert.

3. Procédé d'exécution d'un transfert d'une unité mobile entre une station de base source et une station de base cible selon la revendication 1, dans lequel les paramètres de commande de puissance en liaison montante comprennent un niveau d'interférence en liaison montante au niveau de la station de base cible.

4. Procédé d'exécution d'un transfert d'une unité mobile entre une station de base source et une station de base cible selon la revendication 1, dans lequel les paramètres de commande de puissance en liaison montante comprennent un rapport signal sur interférence plus bruit, SINR, cible au niveau de la station de base cible.

5. Procédé d'exécution d'un transfert d'une unité mobile entre une station de base source et une station de base cible selon la revendication 1, dans lequel les paramètres de commande de puissance en liaison montante comprennent des paramètres de commande de puissance fractionnelle au niveau de la station de base cible.

6. Procédé d'exécution d'un transfert d'une unité mobile entre une station de base source et une station de base cible selon la revendication 1, le procédé comprenant en outre l'étape de transmission d'une mesure d'affaiblissement de trajet entre l'unité mobile et la station de base cible à partir de l'unité mobile.

7. Procédé d'exécution d'un transfert d'une unité mobile entre une station de base source et une station de base cible selon la revendication 6, comprenant en outre l'étape d'utilisation de la mesure d'affaiblissement de trajet lors d'une transmission entre l'unité mobile et la station de base cible.

8. Procédé d'exécution d'un transfert d'une unité mobile entre une station de base source et une station de base cible selon la revendication 1, le procédé comprenant en outre l'étape de transmission entre l'unité mobile et la station de base cible en utilisant les paramètres de commande de puissance en liaison montante.

9. Procédé d'exécution d'un transfert d'une unité mobile entre une station de base source et une station de base cible selon la revendication 1, le procédé comprenant en outre l'étape de transmission entre l'unité mobile et la station de base cible en utilisant les paramètres de commande de puissance en liaison montante et une mesure d'affaiblissement de trajet entre l'unité mobile et la station de base cible.
